# EUROPÄISCHE PATENTSCHRIFT

(11) **EP 2 660 602 B1**
(45) Veröffentlichungstag und Bekanntmachung des Hinweises auf die Patenterteilung: **18.04.2018**
(21) Anmeldenummer: 13165884.1
(22) Anmeldetag: 30.04.2013
(51) Int. Cl.: G01N 33/543, B01L 3/00

(54) **Testkit für die Labordiagnostik**
Test kit for lab diagnostics
Kit de test pour diagnostic de laboratoire

(30) Priorität: 03.05.2012 DE 202012004404 U
(43) Veröffentlichungstag der Anmeldung: 06.11.2013
(73) Patentinhaber: Euroimmun Medizinische Labordiagnostika AG, 23560 Lübeck (DE)
(72) Erfinder: Stöcker, Winfried, 23627 Groß Grönau (DE); Meyer, Wolfgang, 23689 Pansdorf (DE); Scheper, Thomas, 23919 Berkenthin (DE); Euken, Antje, 23564 Lübeck (DE)

(56) Entgegenhaltungen:
- EP-A1- 0 720 020
- DE-U1- 20 215 268
- DE-U1- 29 900 424
- US-A1- 2007 237 687
- US-A1- 2010 025 266

## Beschreibung

Auf dem Gebiet der medizinischen Labordiagnostik sind unterschiedliche Testsysteme bekannt, mit denen Patientenproben auf das Vorhandensein spezieller Antikörper überprüft werden. Durch den Nachweis spezieller Patientenantikörper und deren Zugehörigkeit zu bestimmten Immunglobulinklassen ist es möglich, sowohl Rückschlüsse auf eine bereits stattgefundene oder bestehende bakterielle oder virale Infektion als auch über den zeitlichen Verlauf einer Infektion zu machen. Üblicherweise erfolgt der Nachweis von Antikörpern im Wege der sogenannten Stufendiagnostik, bei der zunächst ein empfindliches Screening und dann eine spezifische Bestätigung durchgeführt werden. In der Routineserologie werden hierbei für das Screening oftmals ELISA (Enzyme-linked immunosorbent assay) verwendet, während als Bestätigungstest vornehmlich Immuno-Blot-Streifen, insbesondere Western-Blot-Streifen, Dot-Blot-Streifen oder Linien-Blot-Streifen eingesetzt werden.

Zur Inkubation der verschiedenen bekannten Immunoblotstreifen werden üblicherweise sogenannte Blotwannen oder Trays verwendet, die eine Mehrzahl von Rinnen aufweisen, in die im Labor die jeweils benötigten Blotstreifen eingelegt werden. Innerhalb der Rinnen findet die Inkubation statt, indem Reagenzien in der hierfür vorgesehenen Reihenfolge in die jeweiligen Rinnen mit den Blotstreifen eingefüllt werden. Im Folgenden wird die Wanne bevorzugt in eine Schwenkbewegung versetzt, so dass eine gute Durchmischung der Reagenzien und eine hinreichende Inkubation der Blotstreifen erfolgen. Nach der Inkubation werden die Streifen zunächst mit Hilfe einer Waschflüssigkeit gereinigt und anschließend die Bandenmuster der einzelnen Streifen ausgewertet. Die Auswertung wird in der Regel automatisiert durchgeführt, wobei die inkubierten Blotstreifen mit einer Kamera oder einem Scannersystem aufgenommen und die aufgenommenen Bilder unter Zuhilfenahme einer Laborsoftware ausgewertet werden.

In diesem Zusammenhang ist aus der DE 202 15 268 U1 eine Blotmembran bekannt, die auf der Rückseite mit einem reversiblen Kleber versehen ist. Der reversible Kleber erlaubt in diesem Fall, die Membran unmittelbar am Boden einer Inkubationsrinne zu fixieren, wodurch ein Umdrehen der Membran durch das Befüllen, Entleeren oder Schwenken der Inkubationsrinne bzw. der Inkubationswanne im Verlauf der Inkubation zuverlässig verhindert wird. Der reversible Kleber wird hierbei wahlweise über die gesamte Ausdehnung der entsprechenden Blotmembranrückseite oder lediglich in einem begrenzten Bereich aufgetragen.

Weiterhin offenbart die DE 202 15 270 U1 ganz spezielle Immunoblotstreifen, auf denen unterschiedliche Arten von Teststreifen vorgesehen sind. Hierbei werden vor allem Ausschnitte von Westernblotstreifen mit Linienblots, durch die jeweils ein spezifisch ausgewähltes Antigen nachweisbar ist, kombiniert.

Ausgehend von den aus dem Stand der Technik bekannten Blotstreifen und den hierfür zur Verfügung stehenden Mitteln zur Inkubation der Streifen liegt der Erfindung die Aufgabe zugrunde, eine Möglichkeit zu schaffen, durch die Untersuchungen mit Hilfe von Blotstreifen komfortabel und gleichzeitig besonders flexibel durchgeführt werden können. Insbesondere die Kombination unterschiedlicher Antikörpernachweise und/oder die gleichzeitige Untersuchung verschiedener Patientenseren während eines gemeinsamen Inkubationsvorgangs soll auf einfache Weise ermöglicht werden. Weiterhin soll sich die anzugebende Vorrichtung dadurch auszeichnen, dass die Lagerung, der Transport und insbesondere die Bereitstellung der einzelnen Blotstreifen zuverlässig und sehr bedienerfreundlich erfolgen können. Vor allem soll es dem Nutzer ermöglicht werden, Inkubationsgänge durchzuführen, bei denen auf einfache Weise unterschiedliche Blotstreifen miteinander kombiniert werden können. Der Aufwand für eine Kombination unterschiedlicher Blotstreifen soll minimiert werden. Weiterhin soll mit verhältnismäßig einfachen Mitteln sichergestellt werden, dass die Inkubation der Immunoblotstreifen effektiv und zuverlässig erfolgt, wobei insbesondere ein Aufschwimmen oder Verdrehen der Teststreifen mit einfachen Mitteln verhindert werden soll.
Die vorstehende Aufgabe wird mit einer Vorrichtung gemäß Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.
Erfindungsgemäß ist eine Vorrichtung zur Inkubation eines Immunoblotstreifens mit wenigstens einer Inkubationsrinne, in die ein länglicher, auf seiner Vorderseite über wenigstens ein biologisches Material verfügender Immunoblotstreifen mit seiner Rückseite einem Rinnenboden zugewandt eingelegt ist, und mit einem Schwenkmittel, durch das der Immunoblotstreifen während der Inkubation wenigstens zeitweise um eine Streifenquerachse schwenkbar ist, derart weitergebildet worden, dass zur Handhabung und/oder Gruppierung von Inkubationsrinnen mit eingelegten Immunoblotstreifen ein Gestell zur Aufnahme wenigstens zweier Inkubationsrinnen vorgesehen ist, und das Gestell über Kraftübertragungspunkte mit dem Schwenkmittel in Wirkverbindung steht. Das Gestell verfügt dabei über parallel nebeneinander angeordnete Aufnahmen, die wenigstens bereichsweise durch Stege voneinander getrennt sind, und zumindest eine Inkubationsrinne zwischen benachbarten Stegen fixiert und zerstörungsfrei aus der Aufnahme lösbar ist. Mit Hilfe der erfindungsgemäßen technischen Lösung ist es auf verhältnismäßig einfache Weise möglich, unterschiedliche Inkubationsrinnen, die bevorzugt bereits mit einem Immunoblotstreifen bestückt sind, zu einer Gruppe zusammenzustellen, die gemeinsam bewegt und inkubiert wird. Besonders vorteilhaft ist die Verwendung eines Gestells, dass in Form eines Rahmens oder Tabletts ausgeführt ist, wobei der Rahmen oder das Tablett definierte Plätze zur Aufnahme der Inkubationsrinnen aufweist. Die für eine Inkubation benötigten Inkubationsrinnen können so bedarfsgerecht in einem Rahmen oder auf einem Tablett angeordnet werden, wobei die Inkubationsrinnen an ihren Plätzen durch geeignete Arretierungshilfen, etwa in Form von Stegen, zuverlässig gehalten werden. Insbesondere bei der Inkubation von Immunoblotstreifen mit Hilfe von Laborautomaten ist das erfindungsgemäß vorgesehene Gestell, das einerseits der Aufnahme der Inkubationsrinnen dient und andererseits schwenkbar ausgeführt und angeordnet ist, besonders vorteilhaft einsetzbar. In diesem Fall können auf besonders vorteilhafte Weise unterschiedliche Inkubationsrinnen bzw. Inkubationsrinnen, die bereits mit unterschiedlichen Blotstreifen bestückt sind, bedarfsgerecht zu einer Gruppe zusammengestellt und in einem hierfür vorgesehenen rahmenförmigen Gestell oder auf einer Ablage eines Laborautomaten zur Inkubation abgestellt werden.

In einer bevorzugten Ausführungsform der Erfindung ist in der Inkubationsrinne ein Halteelement zur Fixierung des Blotstreifens vorgesehen. Das Halteelement ist derart ausgeführt, dass der Immunoblotstreifen sicher in seiner ursprünglich vorgesehenen Position und Lage gehalten wird und insbesondere ein Herausfallen, Verdrehen, Aufschwimmen oder jede ähnliche Bewegung während der Inkubation zuverlässig vermieden wird. Der Immunoblotstreifen wird so sicher innerhalb der Inkubationsrinne fixiert. Auf vorteilhafte Weise ist dieses Halteelement als Rastnase bzw. in Art einer Halteklammer im Bereich des Rinnenbodens ausgeführt. Während der Bestückung der Inkubationsrinne mit einem Immunoblotstreifen wird dieser bereichsweise unter eine derartige Rastnase oder Halteklammer geschoben und auf diese Weise innerhalb der Rinne fixiert.
Bevorzugt ist eine Mehrzahl von Rastnasen im Bereich des Rinnenbodens vorgesehen, so dass ein Blotstreifen in diesem Bereich eingeklemmt und so fixiert werden kann, ohne diesen zu beschädigen. Ebenso ist es denkbar, dass das Halteelement in Form einer Spange ausgeführt ist, an der ein Blotstreifen wenigstens abschnittsweise zu befestigen ist. In jedem Fall ist es sinnvoll, wenn das Halteelement so ausgeführt ist, dass ein Blotstreifen leicht und zerstörungsfrei der Inkubationsrinne entnommen werden kann. Insbesondere nach der Inkubation aber auch sofern ein Bestückungsfehler bemerkt wird, ist dies von großer Bedeutung.

Ergänzend ist es denkbar, dass auf der Rückseite eines Immunoblotstreifens ein Klebstoff vorgesehen ist. Bevorzugt handelt es sich bei dem Klebstoff um einen Haftklebstoff, der ein zumindest nahezu rückstandloses Lösen und erneutes Festkleben des Blotstreifens ermöglicht. Auf vorteilhafte Weise stellt ein derartiger Haftklebstoff auf der Rückseite des Blotstreifens einerseits sicher, dass auch während der Inkubation ein Verrutschen, Verdrehen und / oder Aufschwimmen des Blotstreifens zuverlässig verhindert wird und andererseits, dass der Blotstreifen später zur Auswertung des Tests auf einfache Weise der Inkubationsrinne entnommen werden kann. Hierbei ist der Klebstoff bevorzugt flüssigkeitsbeständig, so dass ein ungewolltes Lösen des Blotstreifens von der Wand der Inkubationsrinne auch während und nach einer Inkubation zuverlässig vermieden wird.
Ferner ist es denkbar, den der Inkubationsrinne entnommenen Teststreifen mit Hilfe des auf der Rückseite vorgesehenen Haftklebstoffes auf einer anderen Oberfläche als dem Rinnenboden zu fixieren. Auf geeignete Weise werden derartige Blotstreifen zur Auswertung auf Auswertebögen in vorgegebener Reihenfolge aufgeklebt, um die Oberfläche der Blot-Streifen abzuscannen und einer elektronischen Laborsoftware zur Auswertung und Diagnoseerstellung durch einen Arzt zuzuleiten.
In einer weiteren speziellen Ausführungsform der Erfindung ist ein Abdeckelement, insbesondere eine Kunststofffolie, vorgesehen, das die Inkubationsrinne mit dem darin befindlichen Blotstreifen zumindest zeitweise gegenüber der Umgebung abschließt. Ferner ist das Abdeckelement gemäß einer Weiterbildung wenigstens bereichsweise an der Inkubationsrinne befestigt und überdeckt diese derart vollumfänglich, dass ein Innenraum der Inkubationsrinne mit dem darin befindlichen Blot-Streifen gegenüber der Umgebung abgeschlossen ist.
Alternativ oder in Ergänzung ist eine Umverpackung, beispielsweise in Form eines Beutels oder Päckchens, vorgesehen, in der die Inkubationsrinne mit dem darin fixierten Blotstreifen aufbewahrt und/oder transportiert wird. Zur Trockenhaltung der Immunoblotstreifen ist die Umverpackung wahlweise derart ausgeführt, dass der Immunoblotstreifen gemeinsam mit einem Trockenmittel durch eine Folie Luftdicht eingeschlossen wird oder aber dass der Blotstreifen mit Hilfe einer wasserdampfdurchlässigen Folie abgeschlossen wird und sich das Trockenmittel auf der dem Blotstreifen gegenüber liegenden Seite der Folie befindet.
Vorzugsweise ist die Umverpackung luftdicht ausgeführt. Eine derartige technische Lösung zeichnet sich dadurch aus, dass die Inkubationsrinne mit dem darin jeweils angeordneten Blot-Streifen eine gegenüber der Umgebung abgeschlossene Einheit bildet.
In einer ganz speziellen Ausführungsform der Erfindung ist vorgesehen, dass die Inkubationsrinnen auf geeignete Weise stapelbar ausgeführt sind. Vorzugsweise können wenigstens zwei Rinnen in einander gestapelt werden, so dass die obere Inkubationsrinne wenigstens bereichsweise in die untere Rinne hineinrutscht. Die Rinnen weisen gemäß einer geeigneten Weiterbildung eine konische Kontur auf, so dass die obere Rinne den Blotstreifen in der unteren Rinne nicht berührt, nachdem die obere in die untere Rinne hineingerutscht ist. Bevorzugt sind die Inkubationsrinnen ferner derart ausgeführt, dass nach dem Stapeln die obere Rinne in Bezug auf die untere Rinne eine Abdeckung bildet. Der in der unteren Inkubationsrinne fixierte Blotstreifen wird in diesem Fall durch die obere Inkubationsrinne gegenüber der Umgebung abgeschlossen.

Gemäß der Erfindung sind wenigstens zwei parallel zueinander angeordnete längliche Inkubationsrinnen vorgesehen, die an ihren Längsseiten einen Verbindungsbereich aufweisen. Hierbei ist es denkbar, dass es sich bei dem Verbindungsbereich um eine geschlossene Fläche handelt oder aber lediglich abschnittsweise Verbindungsstege vorgesehen sind. Auf besonders bevorzugte Weise ist es denkbar, dass der Verbindungsbereich zwischen zwei Inkubationsrinnen eine Perforation, eine Sollbruchstelle, eine Materialschwächung und/oder eine Kerbe aufweist. Ein derart ausgeführter Verbindungsbereich stellt sicher, dass die wenigstens zwei über den Verbindungsbereich miteinander verbundenen Inkubationsrinnen einfach voneinander zu trennen sind, ohne dass hierbei die Inkubationsrinnen beschädigt werden. In diesem Zusammenhang ist es besonders wichtig, dass eine Beschädigung des wannenförmigen Bereichs der Inkubationsrinne, in dem die Inkubation des Blotstreifens stattfindet, zuverlässig ausgeschlossen wird.
Enthält eine Verpackungseinheit eine Mehrzahl von Inkubationsrinnen, die jeweils mit einem Blotstreifen gefüllt, von einem Abdeckelement überdeckt und über einen Verbindungsbereich mit einander verbunden sind, so ist es aufgrund einer geeigneten Gestaltung des Verbindungsbereiches möglich, einzelne oder Gruppen von Immunoblotstreifen der Verpackungseinheit zu entnehmen, ohne dass eine Inkubationsrinne oder ein Abdeckelement derart beschädigt wird, dass ein Blotstreifen ungewollt mit der Umgebung in Kontakt gebracht wird. Insbesondere wird auf diese Weise eine besonders einfache Bereitstellung und Portionierung der benötigten Immunoblotstreifen gewährleistet.
Auf bevorzugte Weise erfolgt die Abtrennung wenigstens einer Inkubationsrinne von einer benachbarten Inkubationsrinne, indem auch die die benachbarten Inkubationsrinnen jeweils überdeckenden Abdeckelemente von einander getrennt werden. Vorzugsweise verfügt in diesem Fall auch das Abdeckelement in einem Verbindungsbereich zwischen den Längsseiten zweier benachbarter Inkubationsrinnen über eine Perforation, eine Sollbruchstelle, eine Materialschwächung und/oder eine Kerbe. Alternativ ist es denkbar, dass jede Inkubationsrinne, gleichgültig ob sie mit einer anderen benachbarten Rinne verbunden ist, über ein separates Abdeckelement verfügt, wobei die Abdeckelemente benachbarter Inkubationsrinnen in diesem speziellen Fall nicht miteinander verbunden sind.
Eine spezielle Weiterbildung der Erfindung sieht ferner vor, dass die Verbindung bzw. der Verbindungsbereich zwischen zwei benachbarten Inkubationsrinnen ausschließlich durch Abdeckelemente gebildet wird, die die Inkubationsrinnen mit den darin befindlichen Blotstreifen überdecken. Bevorzugt ist es denkbar, dass die Abdeckelemente benachbarter Inkubationsrinnen einstückig ausgeführt sind und im Verbindungsbereich zwischen den Inkubationsrinnen eine Perforation, eine Sollbruchstelle, eine Materialschwächung und/oder eine Kerbe vorgesehen ist, um die benachbarten Inkubationsrinnen auf geeignete Weise von einander zu trennen. Vorzugsweise handelt es sich bei dem Abdeckelement um eine Folie, bevorzugt um eine Kunststofffolie, die wenigstens zwei benachbarte Inkubationsrinnen überdeckt. Wiederum sind die Inkubationsrinnen mit den darin befindlichen Blotstreifen zuverlässig gegenüber der Umgebung abgegrenzt, so dass insbesondere eine Verunreinigung, Beschädigung und/oder unzulässige Befeuchtung des Blotstreifens vermieden wird.

Gemäß einer weiteren speziellen Ausführungsform der Erfindung ist an einer Längsseite der Inkubationsrinne wenigstens ein Befestigungselement vorgesehen, über das die Inkubationsrinne mit dem Aufnahmegestell, insbesondere in Form eines Rahmens oder Tabletts, einer weiteren Inkubationsrinne, einer Handhabungseinrichtung, einem Inkubationsautomaten und/oder einer Analysevorrichtung verbindbar ist. In diesem Zusammenhang ist es denkbar, dass wenigstens abschnittsweise im Umfangsbereich einer Inkubationsrinne eine Auflagefläche vorgesehen ist, so dass diese Rinne besonders einfach mittels einer rahmen- oder tablettförmigen Aufnahme, in einer Handhabungseinrichtung, einem Inkubationsautomaten und/oder in einer Analysevorrichtung platzierbar ist. Weiterhin sieht eine spezielle Ausführungsform vor, dass das Befestigungselement über wenigstens eine Schnapp- und/oder Clip-Verbindung verfügt. Wesentlich an der Ausführung des Befestigungselementes ist, dass die Inkubationsrinne auf verhältnismäßig einfache Weise und insbesondere ohne Zuhilfenahme eines Werkzeugs mit einer der vorgenannten Vorrichtungen, insbesondere dem Aufnahmegestell, verbindbar ist.
In einer ganz speziellen Ausführungsform der Erfindung wird eine Inkubationsrinne und/oder eine Inkubationswanne, die wenigstens zwei erfindungsgemäß ausgeführte Inkubationsrinnen aufweist, in einem rahmen- oder tablettförmigen Aufnahmegestell eingesetzt, indem der oder die Randbereiche der Rinnen auf entsprechende Auflagen, bspw. den oberen Begrenzungsflächen der Zwischenstege platziert werden. Bevorzugt erfolgt eine Inkubation bei wenigstens zeitweiser Schwenkbewegung und/oder eine Auswertung während sich die Inkubationsrinne oder die Inkubationsrinnen innerhalb des Aufnahmegestells befinden.
Gemäß einer weiteren Weiterbildung ist es denkbar, dass mit Hilfe eines geeigneten Befestigungselementes eine Inkubationsrinne mit wenigstens einer weiteren Inkubationsrinne verbindbar ist, indem diese im Bereich der Befestigungselemente zusammengesteckt werden. Diese technische Variante bietet die Möglichkeit, eine unterschiedliche Anzahl und/oder sogar unterschiedliche Teststreifen, also Inkubationsrinnen mit unterschiedlichen Blotstreifen, für einen Testlauf bzw. einen Inkubations- und/oder Waschschritt miteinander zu verbinden und auf oder in einem schwenkbar angeordneten Gestell zu platzieren.
In einer speziellen Ausführungsform der Erfindung weist das Antigen, das auf dem Blotstreifen vorgesehen ist, ein biologisches Molekül auf, das sich mittels einer Bindung an einen Antikörper spezifisch nachweisen lässt. Vorzugsweise ist als Antigen ein Protein, ein Lipid, eine Nukleinsäure (DNA/RNA), ein Kohlenhydrat, ein komplexes Molekül und/oder ein Allergen vorgesehen. Die Art des biologischen Materials, das als Antigen auf dem Blotstreifen verwendet wird, richtet sich in erster Linie nach der Untersuchung eines Patienten, die mit Hilfe einer geeigneten In-vitro-Untersuchung einer Körperflüssigkeit des Patienten durchgeführt werden soll.

Bevorzugt handelt es sich bei dem Blotstreifen um einen Linienblot- und/oder einen Westernblotstreifen. Die Erfindung eignet sich insbesondere für Testläufe, bei denen eine Mehrzahl von Inkubationsrinnen mit Immunoblotstreifen in oder auf einem rahmen- oder tablettförmigen Gestell zusammengefasst wird. Durch eine Zusammenfassung mehrerer Inkubationsrinnen mit eingelegten Blotstreifen sind auf besondere Weise Effektivitätssteigerungen bei der Bereitstellung, Inkubation und Auswertung von Immunoblotstreifen möglich.

Im Folgenden wird die Erfindung ohne Beschränkung des allgemeinen Erfindungsgedankens unter Bezugnahme auf die Figuren näher erläutert. Es zeigen:
- Fig. 1:: Vorrichtung zur Inkubation von Immunoblotstreifen mit einer Schwenkeinrichtung und einem tablettförmigen Gestell zur Aufnahme der Inkubationsrinnen;
- Fig. 2:: Perspektivansicht mehrere einzelner Inkubationsrinnen;
- Fig. 3:: Zur Aufnahme einer oder mehrere Inkubationsrinnen geeignetes Tablett;
- Fig. 4:: Teilweise mit Inkubationsrinnen bestücktes Tablett sowie
- Fig. 5:: Vollständig mit Inkubationsrinnen bestücktes Tablett.

Mit der im Folgenden näher erläuterten technischen Lösung lässt sich auf verhältnismäßig einfache Weise eine effektive und zeitsparende Gruppierung, Bereitstellung, Handhabung und Inkubation einer Mehrzahl von Immunoblotstreifen 6 ermöglichen.

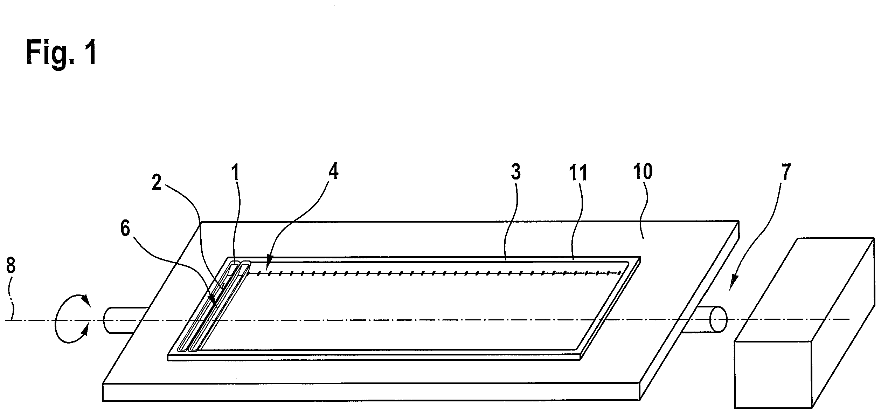

In Figur 1 ist eine Vorrichtung zur Inkubation von Immunoblotstreifen 6 dargestellt. Wesentlich an dieser Vorrichtung ist eine Schwenkeinrichtung 7 mit einer Plattform 10, auf der ein tablettförmiges Gestell 3 angeordnet ist, das seinerseits über nebeneinander angeordnete Aufnahmen 4 verfügt, in die Inkubationsrinnen 1, auf bevorzugte Weise vorbestückte Inkubationsrinnen 1, einlegbar sind. Während einer Inkubation werden die länglichen Inkubationsrinnen 1 wenigstens zeitweise um ihre Querachse 8 geschwenkt, so dass die darin befindlichen Immunoblotstreifen 6 in innigen Kontakt mit dem jeweils in der Rinne 1 befindlichen Reagens gebracht werden. Die einzelnen Aufnahmen 4 des tablettförmigen Gestells 3 sind durch Stege 9 getrennt, zwischen denen die einzelnen Inkubationsrinnen 1 sicher fixiert sind. Die Schwenkeinrichtung 7 verfügt über eine schwenkbare Plattform 10, auf der das tablettförmige Gestell 3 mit den Inkubationsrinnen 1 abgestellt wird, so dass gemeinsam mit der Plattform 10 auch das tablettförmige Gestell 3, die Inkubationsrinnen 1 sowie die darin befindlichen Immunoblotstreifen 6 während einer Inkubation geschwenkt werden können. Um die einzelnen Immunoblotstreifen 6 sicher innerhalb der Inkubationsrinnen 1 zu fixieren und so insbesondere ein Verrutschen, Verdrehen sowie Aufschwimmen der Streifen zu verhindern, sind an den Rinnenböden 2 der Inkubationsrinnen 1 Halteelemente 5 in Form von Spangen vorgesehen, unter denen die Streifen 6 festgeklemmt werden können. Die Klemmung erfolgt derart, dass die Inkubationsstreifen 6 nach einer Inkubation oder in einem anderen Bedarfsfall der Inkubationsrinne 1 entnehmbar sind, ohne dass die Streifen 6 beschädigt werden.

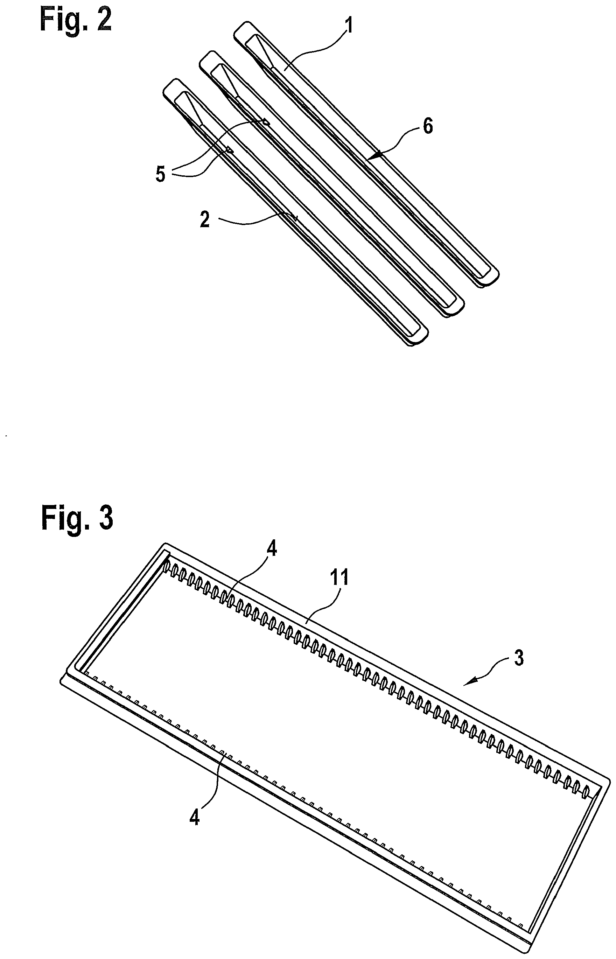

Figur 2 zeigt ferner in einer perspektivischen schrägen Draufsicht drei parallel nebeneinander angeordnete Inkubationsrinnen 1. Die Rinnen 1 sind derart ausgeführt, dass Immunoblotstreifen 6, die bevorzugt auf ihrer Rückseite einen Haftkleber aufweisen, einerseits sicher und andererseits wieder leicht lösbar fixiert werden können. Die Fläche des Rinnenbodens 2 ist nur unwesentlich größer als die Oberfläche der jeweils eingelegten Immunoblotstreifen 6. Auf diese Weise wird sichergestellt, dass die für eine optimale Inkubation benötigte Menge eines Reagens und/oder einer Waschflüssigkeit minimiert werden kann.

An der in dieser Ansicht oberen Stirnseite der Inkubationsrinnen 1 ist die Rinnenwand im Vergleich zur gegenüber liegenden Seite etwas abgeflacht um eine verhältnismäßig einfache Inkubation, insbesondere eine komfortable Einbringung von Fluid in die Rinnen 1, zu ermöglichen. Ferner sind an einer Stelle im Bereich des Rinnenbodens 2 als Halteelemente 5 kleine Spangen oder Rastnasen an die Rinnen 1 angeformt. Diese Spangen bzw. Rastnasen stellen sicher, dass in die Inkubationsrinnen 1 eingelegte Blotstreifen 6 an den Nasen 5 "einrasten" und so effektiv ein späteres Verrutschen, Verdrehen und/oder Aufschwimmen der Streifen 6 während der Inkubation verhindert wird. Die Streifen 6 können so sicher fixiert werden und zwar unabhängig davon, ob sich ein Haftkleber auf der Unterseite bzw. Rückseite der Blotstreifen 6 befindet. Dies ermöglicht wiederum, dass bereits vorbestückte Inkubationsrinnen 1, also Rinnen, die einen Immunoblotstreifen 6 aufweisen, beim Hersteller produziert und so ausgeliefert werden können. In diesem Fall entfällt später im Labor der Arbeitsschritt des Bestückens der Inkubationsrinnen 1 mit den Immunoblotstreifen 6.

An der nach oben offenen Seite der Inkubationsrinnen 1 verfügen diese über einen flachen Rand der die Rinnen 1 an ihrem Umfang vollständig umgibt. Durch das Vorsehen eines derartigen Randes lassen sich die entsprechenden Rinnen 1 leicht und präzise in entsprechende Aufnahmen 4 des Gestells 3 einsetzen und so unmittelbar oder mittelbar mit Handhabungseinrichtungen, Inkubationsautomaten, Auswerteeinheiten oder anderen Laboreinrichtungen verbinden. Die Ränder der Inkubationsrinnen 1 liegen dann auf geeigneten Auflageflächen auf, so dass die jeweilige Rinne 1 sicher gehalten wird, selbst wenn diese, insbesondere während der Inkubation bewegt, bspw. geschwenkt, wird.

In Ergänzung zu den in Figur 2 dargestellten Inkubationsrinnen 1 zeigt Figur 3 ein auf geeignete Weise ausgeführtes tablettartiges Gestell 3. Dieses Tablett 3, das der Aufnahme wenigstens einer, bevorzugt einer Mehrzahl von Inkubationsrinnen 1 dient, ist wannenförmig ausgeführt und verfügt wiederum über einen flachen, leicht nach außen überstehenden Rand 11, der beispielweise auf einer Auflagefläche eines Laborautomaten aufgelegt werden könnte. Genauso ist es denkbar, ein derartiges tablettartiges Gestell 3 mit seiner Unterseite auf einer Plattform 10 abzustellen, durch die das Gestell 3 sowie die Inkubationsrinnen 1 mit den darin angeordneten Immunoblotstreifen 6 während einer Inkubation geschwenkt werden.

An den Längsseiten des wannenförmigen Tabletts 3 sind kleine Stege 9 vorgesehen. Die Stege 9 sind derart ausgeführt und dimensioniert, dass Inkubationsrinnen 1, wie sie beispielsweise in Figur 2 gezeigt sind, in die Zwischenräume 4 zwischen den Stegen 9 einlegbar sind und hierbei eine leichte Klemmung der Rinnen 1 hervorgerufen wird. Auch hier ist wiederum wesentlich, dass die Rinnen 1 einerseits sicher zwischen den Stegen 9 fixiert sind, andererseits aber auch wieder leicht dem Tablett 3 entnommen werden können.

Das Tablett 3 wie auch die Inkubationsrinnen 1 sind aus einem geeigneten Kunststoff ausgeführt, wobei insbesondere das Tablett 3 mehrmals verwendbar ist. Im Bodenbereich ist das in Figur 3 dargestellte Tablett 3 derart ausgeführt, dass es auf einer ebenen Fläche, wie einem Tisch oder einer Ablage eines Laborautomaten abgestellt werden kann. Es versteht sich von selbst, dass Tabletts 3 bedarfsgerecht in unterschiedlichen Größen herstellbar sind, so dass je nach Tablettgröße eine unterschiedliche Anzahl von Inkubationsrinnen 1 und/oder Inkubationswannen, die wenigstens zwei verbundene Inkubationsrinnen 1 aufweisen, auf dem Tablett 3 angeordnet werden können. In Bezug auf einen Laborautomaten ist es wiederum denkbar, dass gleiche oder unterschiedliche Tabletts 3 in den entsprechenden Befestigungsgestellen, Aufnahmerahmen und/oder auf hierfür vorgesehenen Ablageplatten platziert werden.

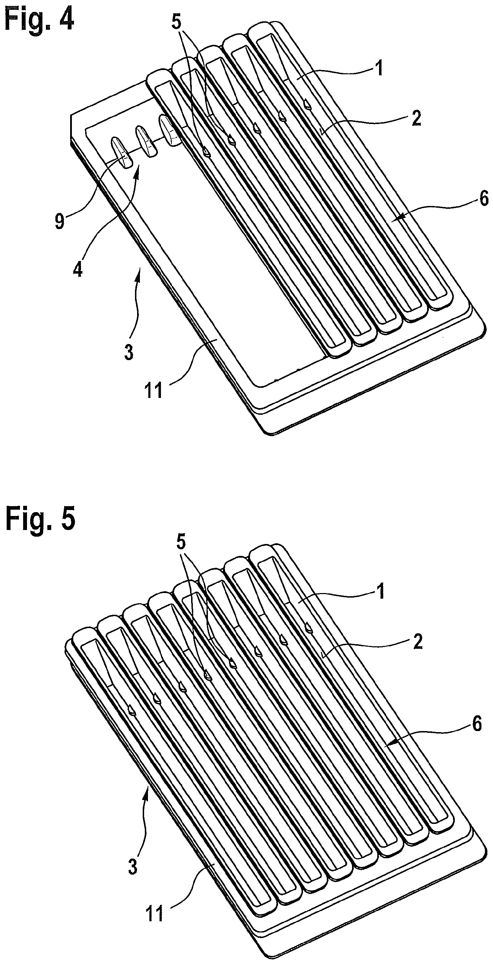

Figur 4 zeigt ein wannenförmiges Tablett 3 mit den im Zusammenhang mit Figur 3 erläuterten Stegen 9 und einem umlaufenden Rand 11 an der Außenseite, das mit fünf einzelnen Inkubationsrinnen 1 bestückt ist. Die Rinnen 1 wurden in die hierfür vorgesehenen Aufnahmen 4 eingelegt und sind durch die Stege 9 fixiert. In dem dargestellten Ausführungsbeispiel sind drei weitere Aufnahmebereiche 4, die zur Fixierung von Inkubationsrinnen 1 geeignet sind, nicht mit Rinnen 1 bestückt worden.

Innerhalb der einzelnen Inkubationsrinnen 1 sind im Bodenbereich 2 gegenüberliegende Spangen 5 vorgesehen, durch die ein in die Rinnen 1 eingelegter Immunoblotstreifen 6 sicher am Boden gehalten wird und so ein Verrutschen, Verdrehen und/oder ein Aufschwimmen während der Inkubation verhindert wird. Die Anzahl sowie die Ausführung und Anordnung der einzelnen Spangen 5 wird stets derart gewählt, dass eine sichere Fixierung der Immunoblotstreifen 6 in den Rinnen 1 gewährleistet wird. Selbstverständlich ist es denkbar, die Rinnen 1 bereits vor der Auslieferung in die Labore mit Blotstreifen 6 zu bestücken und/oder die Inkubationsrinnen 1 bereits in dem Tablett 3 zu platzieren.

Im Vergleich zu Figur 4 zeigt Figur 5 ein wannenförmiges Tablett 3, das vollständig mit Inkubationsrinnen 1, in dem dargestellten Beispiel sind es acht Stück, bestückt worden ist. Die Inkubationsrinnen 1 sind hierbei sicher innerhalb der Wanne des Tabletts 3 durch die dort vorgesehenen Stege 9 fixiert.

Die in den Figuren 3, 4 und 5 dargestellten Tabletts 3 ermöglichen zum einen auf besonders einfache Weise eine Gruppierung unterschiedlicher Immunoblotstreifen 6 und zum anderen eine sichere Handhabung, insbesondere Transport, Fixierung in einem Laborautomaten, Inkubation und Auswertung, einer Mehrzahl von Inkubationsrinnen 1 mit den hierin angeordneten Blotstreifen 6. Generell können so eine Vielzahl von gleichen und/oder unterschiedlichen Immunoblotstreifen 6 gleichzeitig bewegt, prozessiert und ausgewertet werden, wobei es wiederum denkbar ist, dass die Rinnen 1 vereinzelt oder mit einander verbunden sind. Ebenso ist es denkbar, die Inkubationsrinnen 1 oder eine Mehrzahl von Rinnen 1 zu stapeln und/oder diese zu Verpackungseinheiten zusammen zu fassen. Verpackungseinheiten können hierbei gebildet werden, indem eine oder eine Mehrzahl von Inkubationsrinnen 1 mit oder ohne Tablett 3 in einer Umverpackung angeordnet oder die Inkubationsrinnen 1 mit den darin angeordneten Blotstreifen 6 mit einer Schutzfolie überdeckt werden.

### Bezugszeichenliste

- 1: Inkubationsrinne
- 2: Rinnenboden
- 3: Gestell
- 4: Aufnahme
- 5: Halteelement
- 6: Immunoblotstreifen
- 7: Schwenkeinrichtung
- 8: Streifenquerachse
- 9: Steg
- 10: Plattform
- 11: Befestigungselement

## Patentansprüche

1. Vorrichtung zur Inkubation eines Immunoblotstreifens (6) mit wenigstens einer Inkubationsrinne (1), in die ein länglicher, auf seiner Vorderseite über wenigstens ein biologisches Material verfügender Immunoblotstreifen (6) mit seiner Rückseite einem Rinnenboden (2) zugewandt eingelegt ist, und mit einem Schwenkmittel (7), durch das der Immunoblotstreifen (6) während der Inkubation wenigstens zeitweise um eine Streifenquersachse (8) schwenkbar ist,
**dadurch gekennzeichnet, dass** zur Handhabung und/oder Gruppierung von Inkubationsrinnen (1) mit eingelegten Immunoblotstreifen (6) ein Gestell (3) zur Aufnahme wenigstens zweier Inkubationsrinnen (1) vorgesehen ist, und das Gestell (3) über Kraftübertragungspunkte mit dem Schwenkmittel (7) in Wirkverbindung steht,
und dass das Gestell (3) über parallel nebeneinander angeordnete Aufnahmen (4) verfügt, die wenigstens bereichsweise durch Stege (9) von einander getrennt sind, und zumindest eine Inkubationsrinne (1) zwischen benachbarten Stegen (9) fixiert und zerstörungsfrei aus der Aufnahme (4) lösbar ist.

2. Vorrichtung nach Anspruch 1,
**dadurch gekennzeichnet, dass** das Gestell (3) Befestigungselemente (11) aufweist, mittels derer das Gestell (3) in einem Aufnahmemittel oder auf einer Plattform (10) eines Laborautomaten platzierbar ist.

3. Vorrichtung nach einem der Ansprüche 1 bis 2,
**dadurch gekennzeichnet, dass** die Inkubationsrinne (1) am Rinnenboden (2) über wenigstens ein Halteelement (5) verfügt, durch das der eingelegte Immunoblotstreifen (6) fixierbar ist.

4. Vorrichtung nach einem der Ansprüche 1 bis 3,
**dadurch gekennzeichnet, dass** der Immunoblotstreifen (6) auf seiner dem Rinnenboden (2) zugewandten Rückseite einen Haftklebstoff aufweist.

5. Vorrichtung nach einem der Ansprüche 1 bis 4,
**dadurch gekennzeichnet, dass** wenigstens eine Inkubationsrinne (1) von einer Folie überdeckt ist.

6. Vorrichtung nach einem der Ansprüche 1 bis 5,
**dadurch gekennzeichnet, dass** wenigstens zwei Inkubationsrinnen (1) vorgesehen sind, die an ihren Längsseite eine Verbindung aufweisen.

7. Vorrichtung nach Anspruch 6,
**dadurch gekennzeichnet, dass** die Verbindung und die wenigstens zwei Inkubationsrinnen (1) einstückig ausgeführt sind.

8. Vorrichtung nach Anspruch 6,
**dadurch gekennzeichnet, dass** die Verbindung durch eine die wenigstens zwei Inkubationsrinnen (1) überdeckende Folie gebildet wird.

9. Vorrichtung nach einem der Ansprüche 6 bis 8,
**dadurch gekennzeichnet, dass** die Verbindung eine Materialschwächung und/oder wenigstens eine Ausnehmung, insbesondere eine Perforation aufweist.

10. Verfahren zur Inkubation eines Immunoblotstreifens (6), bei dem wenigstens ein länglicher Immunoblotsreifen (6), der auf seiner Vorderseite mit einem biologischen Material versehen ist, in eine Inkubationsrinne (1) eingelegt wird, wobei die Rückseite des Immunoblotstreifens (6) einem Rinnenboden (2) zugewandt ist, und bei dem der Immunoblotstreifen (6) während der Inkubation wenigstens zeitweise um eine Streifenquersachse (8) geschwenkt wird,
**dadurch gekennzeichnet, dass** wenigstens zwei Inkubationsrinnen (1) mit darin befindlichen Immunoblotstreifen (6) in hierfür vorgesehene Aufnahmen (4) eines Gestells (3) eingelegt werden und das mit Inkubationsrinnen (1), die mit Immunoblotstreifen (6) bestückt sind, besetzte Gestell (3) derart mit einer Schwenkeinrichtung (7) verbunden wird, dass das Gestell (3) gemeinsam mit den Inkubationsrinnen (1) und den darin befindlichen Immunoblotstreifen (6) während der Inkubation wenigstens zeitweise geschwenkt wird,
wobei das Gestell (3) über parallel nebeneinander angeordnete Aufnahmen (4) verfügt, die wenigstens bereichsweise durch Stege (9) von einander getrennt sind, und zumindest eine Inkubationsrinne (1) zwischen benachbarten Stegen (9) fixiert und zerstörungsfrei aus der Aufnahme (4) lösbar ist.

11. Verfahren nach Anspruch 10,
**dadurch gekennzeichnet, dass** die wenigstens zwei Immunoblotstreifen (6) wenigstens bereichsweise am Rinnenboden (2) der Inkubationsrinne (1) festgeklemmt werden.

12. Verfahren nach Anspruch 10 oder 11,
**dadurch gekennzeichnet, dass** das Gestell (3) auf eine Plattform (10) der Schwenkeinrichtung (7) gestellt wird.

## Claims

1. A device for incubating an immunoblot strip (6) having: at least one incubation channel (1), into which an elongated immunoblot strip (6) is inserted that disposes on its front surface at least one biological material and whose back surface is facing the channel bottom (2), and a swivel means (7), wherein said immunoblot strip (6) is swiveling around a transverse axis (8) at least at times of incubation,
**characterized in that** for handling and/or grouping of incubation channels (1) with inserted immunoblot strips (6) a rack (3) is provided that can store at least two incubation channels (1) and wherein said rack (3) is in operative connection with the swiveling means (7) via force transmission points, and wherein the rack (3) has pick-ups (4) arranged in parallel which are separated from one another at least in certain areas by bridges (9), and wherein at least one incubation channel (1) is fixed between adjacent bridges (9) and is detachable from the pick-ups (4) without being destroyed.

2. The device according to claim 1, wherein said rack (3) has fastening elements (11) for placing said rack (3) in one of said pick-ups or onto a platform (10) of automatic laboratory equipment.

3. The device according to one of claims 1 to 2, wherein said incubation channel (1) has at least one holding element (5) on said channel bottom (2) by which the inserted immunoblot strip (6) can be fixed.

4. The device according to one of claims 1 to 3, wherein said immunoblot strip (6) has an adhesive on its back surface facing the channel bottom (2).

5. The device according to one of claims 1 to 4, wherein said at least one incubation channel (1) is covered by a film.

6. The device according to one of claims 1 to 5, wherein at least two incubation channels (1) are provided having a connection at their respective longitudinal sides.

7. The device according to claim 6, wherein said connection and said at least two incubation channels (1) are designed in one piece.

8. The device according to claim 6, wherein said connection is formed by a film covering said at least two incubation channels (1).

9. The device according to one of claims 6 to 8, wherein said connection has a diminished material strength and/or at least one recess, in particular a perforation.

10. A method for incubating of an immunoblot strip (6), wherein at least one elongated immunoblot strip (6), which disposes a biological material on its front surface, is inserted into an incubation channel (1), wherein the back surface of the immunoblot strip (6) faces a channel base (2), and in which the immunoblot strip (6) is swiveling around a transverse axis (8) at least at times of incubation, **characterized in that** at least two incubation channels (1) with immunoblot strips (6) located therein are inserted into for this purpose designed pick-ups (4) of a rack (3) and wherein the rack (3) equipped with incubation channels (1) that contain immunoblot strips (6) connected to a swivel device (7) such that the rack (3), together with the incubation channels (1) and the immunoblot strips (6) located therein, is swiveled at least temporarily during the incubation,
wherein the rack (3) has pick-ups (4) arranged in parallel which are separated from one another at least in certain areas by bridges (9), and wherein at least one incubation channel (1) is fixed between adjacent bridges (9) said incubation channel being detachable from said pick-ups (4) without being destroyed.

11. The method according to claim 10, wherein said at least two immunoblot strips (6) are fixed at the channel bottom (2) of the incubation channel (1) at least in certain areas.

12. The method according to claim 10 or 11, wherein said rack (3) is positioned on a platform (10) of the swivel device (7).

## Revendications

1. Dispositif pour l'incubation d'une bandelette d'immunotransfert (6) avec au moins un canal d'incubation (1), dans lequel une bandelette d'immunotransfert allongée (6) munie d'au moins un matériau biologique sur son côté avant est posée avec son côté arrière tourné vers le fond de canal (2), et avec un moyen de pivotement (7), au moyen duquel la bandelette d'immunotransfert (6) peut pivoter au moins temporairement autour d'un axe transversal (8) de la bandelette pendant l'incubation, **caractérisé en ce qu'**il est prévu pour la manipulation et/ou le groupage de canaux d'incubation (1) munis de bandelettes d'immunotransfert (6) un châssis (3) destiné à recevoir au moins deux canaux d'incubation (1), et le châssis (3) est en liaison active avec le moyen de pivotement (7) par l'intermédiaire de points de transmission de force,
et **en ce que** le châssis (3) est doté de logements (4) disposés parallèlement l'un à côté de l'autre, qui sont séparés l'un de l'autre au moins localement par des nervures (9), et au moins un canal d'incubation (1) est fixé entre des nervures voisines (9) et peut être séparé du logement (4) sans destruction.

2. Dispositif selon la revendication 1, **caractérisé en ce que** le châssis (3) présente des éléments de fixation (11), au moyen desquels le châssis (3) peut être placé dans un moyen de logement ou sur une plate-forme (10) d'un automate de laboratoire.

3. Dispositif selon une des revendications 1 à 2, **caractérisé en ce que** le canal d'incubation (1) est doté sur le fond de canal (2) d'au moins un élément de maintien (5), par l'intermédiaire duquel la bandelette d'immunotransfert posée (6) peut être fixée.

4. Dispositif selon l'une quelconque des revendications 1 à 3, **caractérisé en ce que** la bandelette d'immunotransfert (6) présente une substance adhésive sur son côté arrière tourné vers le fond de canal (2).

5. Dispositif selon l'une quelconque des revendications 1 à 4, **caractérisé en ce qu'**au moins un canal d'incubation (1) est recouvert par une feuille.

6. Dispositif selon l'une quelconque des revendications 1 à 5, **caractérisé en ce qu'**il est prévu au moins deux canaux d'incubation (1), qui présentent une liaison sur leur côté longitudinal.

7. Dispositif selon la revendication 6, **caractérisé en ce que** la liaison et lesdits au moins deux canaux d'incubation (1) sont réalisés en une seule pièce.

8. Dispositif selon la revendication 6, **caractérisé en ce que** la liaison est formée par une feuille recouvrant lesdits au moins deux canaux d'incubation (1).

9. Dispositif selon l'une quelconque des revendications 6 à 8, **caractérisé en ce que** la liaison présente un affaiblissement de la matière et/ou au moins un évidement, en particulier une perforation.

10. Procédé pour l'incubation d'une bandelette d'immunotransfert (6), dans lequel on pose dans un canal d'incubation (1) au moins une bandelette allongée d'immunotransfert (6), qui est munie d'un matériau biologique sur son côté avant, dans lequel le côté arrière de la bandelette d'immunotransfert (6) est tournée vers un fond de canal (2), et dans lequel on fait pivoter la bandelette d'immunotransfert (6) au moins temporairement autour d'un axe transversal (8) de la bandelette pendant l'incubation, **caractérisé en ce que** l'on dépose au moins deux canaux d'incubation (1) avec des bandelettes d'immunotransfert (6) placées à l'intérieur dans des logements (4) prévus à cet effet d'un châssis (3) et on relie le châssis (3) occupé par des canaux d'incubation (1), qui sont garnis de bandelettes d'immunotransfert (6), à un dispositif de pivotement (7), de telle manière que l'on fasse pivoter le châssis (3) en même temps que les canaux d'incubation (1) et les bandelettes d'immunotransfert (6) placées à l'intérieur au moins temporairement pendant l'incubation,
dans lequel le châssis (3) comporte des logements (4) disposés parallèlement l'un à côté de l'autre, qui sont séparés l'un de l'autre au moins localement par des nervures (9), et au moins un canal d'incubation (1) est fixé entre des nervures voisines (9) et peut être séparé du logement (4) sans destruction.

11. Procédé selon la revendication 10, **caractérisé en ce que** l'on fixe lesdites au moins deux bandelettes d'immunotransfert (6) au moins localement au fond de canal (2) du canal d'incubation (1).

12. Procédé selon la revendication 10 ou 11, **caractérisé en ce que** l'on installe le châssis (3) sur une plate-forme (10) du dispositif de pivotement (7).
